# EUROPEAN PATENT SPECIFICATION

(11) **EP 2 608 765 B1**
(45) Date of publication and mention of the grant of the patent: **16.12.2015**
(21) Application number: 11746244.0
(22) Date of filing: 22.08.2011
(51) Int. Cl.: A61K 8/27, A61K 8/29, A61K 8/40, A61K 8/97, A61Q 1/02, A61Q 17/04, A61Q 19/00, A61Q 19/02, A61K 8/02

(54) **A PHOTOPROTECTIVE PERSONAL CARE COMPOSITION**
KÖRPERPFLEGEZUSAMMENSETZUNG MIT LICHTSCHUTZ
COMPOSITION DE SOINS PERSONNELS PHOTOPROTECTRICE

(30) Priority: 24.11.2010 EP 10192313; 24.08.2010 IN MU23622010
(43) Date of publication of application: 03.07.2013
(73) Proprietor: Unilever NV, 3013 AL Rotterdam (NL); Unilever PLC, London Greater London EC4Y 0DY (GB)
(72) Inventor: RAJAGOPAL, Ramasubramaniam, Whitefield Bangalore 560 066 (IN); ROY, Arindam, Whitefield Bangalore 560 066 (IN)
(74) Representative: Corsten, Michael Allan
(86) International application number: PCT/EP2011/064358
(87) International publication number: WO 2012/025478

(56) References cited:
- EP-A2- 0 266 124
- EP-A2- 1 371 359
- US-A1- 2005 019 285
- US-A1- 2005 042 186

## Description

### Technical Field

The invention relates to photoprotective personal care compositions. The invention more particularly relates to a composition that affords protection against visible light while ensuring an even skin tone when the composition is applied on the skin.

### Background of the invention

People all over the world like to protect their skin against harsh environmental conditions like heat, humidity, sunlight, pollution and dust. In doing so, they not only desire to have healthy skin which appears young but also to have a pleasing skin appearance. In tropical countries where people generally have dark skin, there is a desire to have lighter skin appearance. People who live far from the tropical areas e.g. the Caucasian people who generally have lighter skin, prefer to have an even tanned tone of their skin. Any exposure of their skin to sunlight, often leads to blotchy skin, referred to as freckles and in some cases they experience hyperpigmentation in localized areas of the skin. In all of the above cases, people generally rely on cosmetic solutions to their skin protection and appearance problems.

In providing photoprotection of the skin, manufacturers, heretofore have concentrated mostly on using ingredients which block or absorb ultra-violet (UV) rays of the sun from reaching the skin. Solar radiation includes about 5% UV radiation, wavelength of which is between 200 nm and 400 nm. It is further classified into three regions: from 320 to 400 nm (UV-A), 290 to 320 nm (UV-B) and from 200 to 290 nm (UV-C). The emphasis has been on protection from UV radiation since it is believed to be the cause of skin coloration or tanning and if such tanning is uneven, it is disliked by the consumer. Sunscreens or sunblocks may be organic compounds or inorganic compounds. Sunscreens are generally organic compounds that work by absorbing ultra-violet (UV) radiation from the sun at specified wavelength range thus not permitting the UV radiation from reaching the skin surface. UV sunblocks are generally inorganic compounds that act as physical barrier against a wide range of radiation from the sun (both UV and visible light).

Of late it has been identified that visible light also contributes significantly to tanning e.g. in Effects of Visible Light on the Skin, Photochemistry and Photobiology Volume 84 Issue 2, Pages 450 - 462, 2008*.* Therefore, the present inventors have been working on preparing cosmetic compositions that give protection against visible light without compromising on the other benefits that they are used to. One traditional approach is to use inorganic sunblocks. Inorganic sunblocks, while being broad spectrum, often are white in colour and leave a pale whitish appearance on the skin which is unnatural and not liked by consumers.

In order to provide a solution to the above problem the present inventors have been working intensively on developing a composition that provides photoprotection especially against visible light. They found that in order to deliver this, it is important to have a composition that contains ingredients that have two different properties i.e. (i) one material which ensures reflection of incident light predominantly in the blue region and (ii) another material which ensures absorption of incident light predominantly in the blue region. This unique combination ensures not only photoprotection against visible light but also delivers a well sought after natural tone of the skin without it being unnaturally pale or unnaturally dark. In order to deliver this, the inventors have invented a selective combination of an inorganic pigment particle that has a specific light scattering property and an organic dye that has a specific light absorption property. They found that this is especially effective when used by dark skinned consumers.

Compositions that combine inorganic particles and organic dyes are known. EP 1 371 359 (Biersdorf, 2003), discloses a cosmetic or dermatological composition comprising a titanium or iron oxide pigment with a mean particle diameter above 500 nm, an organic dye, and one or more UV filters selected from asymmetrically substituted triazines, phenylene-1,4-bis(2-benzimidazyl)-3,3',5,5'-tetrasulfonic acid and its salts and 2,2'-methylenebis(6-(2H-benzotriazol-2-yl)-4-(1,1,3,3-tetramethylbutyl)phenol).

US 2002/0176831 (Avon) discloses a coloured cosmetic composition comprising: an emulsion formed of an aqueous phase and an oil phase, said aqueous phase having a hydrophilic colourant which is preferably a dye or an in inorganic particle; and a pearlescent colorant which is preferably selected from a group of inorganic colourant particulates being present in the oil phase or external to the emulsion.

US 6 080 415 (L'Oreal, 2000) discloses a cosmetic make-up product comprising two components packaged separately, the first component having a photochromic colouring agent which is selected from a list of inorganic particles or organic dyes which is capable of producing at least one colour in the presence of UV light and the second component comprising an agent that screens UV light.

While there is general mention of use of inorganic pigments and dyes in the above cited publication, none of the them disclose that a combination of a selective inorganic pigment particle of specified size that has a specific light scattering property (reflection of incident light predominantly in the blue region) and selected organic dyes that have a specific light absorption property (absorption of incident light predominantly in the blue region) provides for enhanced visible light protection while ensuring even skin appearance.

It is thus an object of the present invention to provide for a photoprotective cosmetic composition that gives the benefits of photoprotection over the visible light region while giving the skin a pleasing even appearance.

### Summary of the invention

According to the present invention there is provided a photoprotective personal care composition comprising
(i) 0.5 to 10% of inorganic pigment particles in the size range between 0.015 to 0.4 micrometer which scatter greater than 50% of light in the wavelength range 400 to 550 nm with respect to total scattered light in the range of 400 to 700 nm selected from titanium dioxide or zinc oxide, and
(ii) 0.01 to 0.5% of organic dye which absorbs greater than 50% of light in the wavelength range 400 to 550 nm with respect to total absorbed light in the range of 400 to 700 nm selected from FDC Red 4, FDC Red 1 or DC Brown.

### Detailed description of the invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. The disclosure of the invention as found herein is to be considered to cover all embodiments as found in the claims as being multiply dependent upon each other irrespective of the fact that claims may be found without multiple dependency or redundancy.

"Personal Care Composition" as used herein, is meant to include a composition for topical application to skin of mammals, especially humans. Such a composition is preferably a leave-on composition. It includes any product applied to a human body for getting photoprotection, improving appearance, cleansing, odor control or general aesthetics and is especially useful for protection against harmful effects of light on the skin. The composition of the present invention can be in the form of a liquid, lotion, cream, foam, scrub, gel, or toner, or applied with an implement or via a face mask, pad, patch or as a spray. Non-limiting examples of personal care compositions include leave-on compositions like skin lotions, creams, antiperspirants, deodorants, depilatories, lipsticks, foundations, mascara, sunless tanners and sunscreen lotions. "Skin" as used herein is meant to include skin on the face and body (e.g., neck, chest, back, arms, underarms, hands, legs, buttocks and scalp). The composition is preferably not a wash-off product since in these cases, the particle and dye are not deposited in sufficient amounts on the skin to enable the desired improvement in skin appearance.

The present invention relates to a photoprotective personal care composition. The composition comprises inorganic pigment particles which scatter greater than 50% of light in the wavelength range 400 to 550 nm with respect to total scattered light in the range of 400 to 700 nm. This is to mean that the inorganic pigment particles scatter light more in the blue region of the visible light spectrum (400 to 550 nm) as compared to other regions of the visible light spectrum (550 to 700 nm). Thus, when visible light is incident on the inorganic pigment particles used in the composition of the invention, the light is predominantly scattered in the blue region (400 to 550 nm) of the spectrum. The composition comprises 0.5 to 10%, preferably 1 to 5% by weight of the inorganic pigment particles which are titanium dioxide or zinc oxide. The particle size of the inorganic pigment particles are between 0.015 and 0.4 micrometer, more preferably in the range of more than 0.1 to less than 0.4 micrometer. By particle size is meant particle diameter. It is particularly preferred that the inorganic pigment particles are not coloured i.e the inorganic pigment particles are white in colour, and do not impart colour to the composition..

The composition of the invention comprises an organic dye which absorbs greater than 50% of light in the wavelength range 400 to 550 nm with respect to total absorbed light in the range of 400 to 700 nm. This is to mean that the organic dye absorbs light more in the blue region of the visible light spectrum (400 to 550 nm) as compared to other regions of the visible light spectrum (550 to 700 nm). Thus, when visible light is incident on the organic dye used in the composition of the invention, the light is predominantly absorbed in the blue region (400 to 550 nm) of the spectrum. The composition comprises 0.01 to 0.5%, preferably 0.01 to 0.3% by weight of the organic dye. The organic dyes for use in the composition of the invention are FDC Red 4, FDC Red 1 or DC Brown (e.g. Chocolate Brown TAS). Combinations of these dyes, sometimes available commercially as mixtures are also within the scope of the invention.

It is especially useful for use by consumers who are more dark skinned. By dark skinned is meant those whose skin colour has a L* value of 30 to 65, where L* is the lightness of skin colour in the CIE L*a*b* scale. Without wishing to be bound by theory, it is believed that the combination of the inorganic pigment particle and the organic dye which on the one hand provides the necessary photoprotection against visible light for all category of consumers (light skinned as well as dark skinned), the present invention is especially effective in providing a natural skin tone to dark skinned consumers without giving an unnatural pale skin appearance or an unnaturally dark skin appearance.

A suitable way of incorporating the selective particle and selective dye in the composition of the invention is to mix the two ingredients at suitable steps in process of preparation of the composition. Alternately, it is also possible to prepare the particles in such a way that they are coated with the selected dye. The particles thus coated with the dye could be incorporated in the composition of the invention.

According to a preferred aspect of the invention, an extract of a natural material is incorporated in the composition of the invention. A preferred extract is black tea extract. It is useful to incorporate 0.1 to 5% by weight of tea extract in the composition of the invention and this has been seen to further improve the visible light photoprotection over and above the photoprotection provided by the first aspect of the invention. Inclusion of tea extract ensures that the visible light photoprotection is achieved with no compromise on the desired skin appearance.

The composition of the present invention is preferably delivered to the external surface of the human body which is exposed to visible light e.g. skin or hair through a cosmetically acceptable base. Suitable cosmetically acceptable bases are cream, lotion, gel, emulsion or spray.

Cosmetic compositions may be prepared using different cosmetically acceptable emulsifying or non-emulsifying systems and vehicles. A highly suitable base is a cream. Vanishing creams are especially preferred. Vanishing cream bases generally comprise 5 to 25% by weight fatty acid and 0.1 to 10% by weight soap. Vanishing cream base gives a highly appreciated matty feel to the skin. C₁₂ to C₂₀ fatty acids are especially preferred in vanishing cream bases, further more preferred being C₁₄ to C₁₈ fatty acids. The most preferred fatty acid is stearic acid. The fatty acid in the composition is more preferably present in an amount in the range of 5 to 20% by weight of the composition. Soaps in the vanishing cream base include alkali metal salt of fatty acids, like sodium or potassium salts, most preferred being potassium stearate. The soap in the vanishing cream base is generally present in an amount in the range of 0.1 to 10%, more preferably 0.1 to 3% by weight of the composition. Generally the vanishing cream base in cosmetic compositions is prepared by taking a desired amount of total fatty matter and mixing with potassium hydroxide in desired amounts. The soap is usually formed insitu during the mixing. The composition of the invention preferably comprises water. Water is preferably present in 35 to 90%, more preferably 50 to 85% by weight of the composition.

The composition of the invention may additionally comprise a skin lightening agent. The skin lightening agent is preferably chosen from one or more of one or more of a vitamin B3 compound or its derivative e.g. niacin, nicotinic acid, niacinamide. Any other well known skin lightening agents e.g. aloe extract, ammonium lactate, azelaic acid, kojic acid, butyl hydroxy anisole, butyl hydroxy toluene, citrate esters, 2, 5 dihydroxybenzoic acid and its derivatives, ellagic acid, fennel extract, green tea extract, hydroquinone, 4 hydroxyanisole and its derivatives, 4-hydroxy benzoic acid derivatives, hydroxycaprylic acid, lemon extract, linoleic acid, magnesium ascorbyl phosphate, salicylic acid, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof, may also be incorporated in the composition of the invention. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10%, more preferably 0.2 to 5% by weight of the composition.

The cosmetic composition may preferably additionally comprise one or more UV sunscreens. The UV sunscreens may be inorganic or organic.

A wide variety of organic sunscreen agents are suitable for use in combination with the essential ingredients of this invention. Suitable UV-A / UV-B sunscreen agents include, 2-hydroxy-4-methoxybenzophenone, octyldimethyl-p-aminobenzoic acid, digalloyltrioleate, 2,2-dihydroxy-4-methoxybenzophenone, ethyl-4-(bis(hydroxypropyl)) aminobenzoate, 2-ethylhexyl-2-cyano-3,3-diphenylacrylate, 2-ethylhexylsalicylate, glyceryl-p-aminobenzoate, 3,3,5-trimethylcyclohexylsalicylate, methylanthranilate, p-dimethylaminobenzoic acid or aminobenzoate, 2-ethylhexyl-p-dimethyl-amino-benzoate, 2-phenylbenzimidazole-5-sulfonic acid, 2-(p-dimethylaminophenyl)-5-sulfonicbenzoxazoic acid, 2-ethylhexyl-p-methoxycinnamate, butylmethoxydibenzoylmethane, 2-hydroxy-4-methoxybenzophenone, octyldimethyl-p-aminobenzoic acid and mixtures thereof. Most suitable organic sunscreen are 2-ethylhexyl-p-methoxycinnamate and butylmethoxydibenzoylmethane.

A safe and effective amount of sunscreen may be used in the compositions useful in the subject invention. The composition preferably comprises from about 0.1 % to about 10%, more preferably from about 0.1 % to about 5% by weight of a sunscreen agent.

The composition according to the invention may also comprise other diluents. The diluents act as a dispersant or carrier for other materials present in the composition, so as to facilitate their distribution when the composition is applied to the skin.

Diluents other than water can include liquid or solid emollients, solvents, humectants, thickeners and powders.

The cosmetically acceptable base is usually from 10 to 99.9%, preferably from 50 to 99% by weight of the composition, and can, in the absence of other cosmetic adjuncts, form the balance of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

The composition is formulated in any known format, more preferred formats being creams, lotions or sprays.

The composition of the invention may comprise a conventional deodourant base as the cosmetically acceptable carrier. By a deodorant is meant a product in the stick, roll-on, or propellant medium which is used for personal deodorant benefit e.g. application in the under-arm or any other area which may or may not contain anti-perspirant actives.

Deodorant compositions can generally be in the form of firm solids, soft solids, gels, creams, and liquids and are dispensed using applicators appropriate to the physical characteristics of the composition.

According to yet another aspect of the present invention there is provided use of a composition of the invention for protection of the external surface of a human body against the harmful effects of visible light without altering the skin appearance. The use is preferably non-therapeutic use.

The invention is now further described by way of the following non-limiting examples.

### EXAMPLES

### Example 1 to 6: Transmittance (measure of photoprotection) and reflectance (measure of skin appearance) provided by compositions as per the invention (example 2) as compared to examples outside the invention (examples 1 and 3 to 6)

The following compositions (as shown in table 1) were prepared.

**Table 1**

| Examples | 1 Wt% | 2 Wt% | 3 Wt% | 4 Wt% | 5 Wt% | 6 Wt% |
|---|---|---|---|---|---|---|
| Hysteric acid | 4.0 | 4.0 | 4.0 | 4.0 | 4.0 | 4.0 |
| Glycerine | 1.0 | 1.0 | 1.0 | 1.0 | 1.0 | 1.0 |
| Isopropyl myristate | 3.0 | 3.0 | 3.0 | 3.0 | 3.0 | 3.0 |
| Sodium hydroxide | 0.05 | 0.05 | 0.05 | 0.05 | 0.05 | 0.05 |
| Cetyl alcohol | 0.20 | 0.20 | 0.20 | 0.20 | 0.20 | 0.20 |
| Silicone oil | 0.50 | 0.50 | 0.50 | 0.50 | 0.50 | 0.50 |
| Methyl paraben + propyl paraben | 0.30 | 0.30 | 0.30 | 0.30 | 0.30 | 0.30 |
| Phenoxy ethanol | 0.60 | 0.60 | 0.60 | 0.60 | 0.60 | 0.60 |
| Disodium EDTA | 0.04 | 0.04 | 0.04 | 0.04 | 0.04 | 0.04 |
| Parsol 1789 | 0.4 | 0.4 | 0.4 | 0.4 | 0.4 | 0.4 |
| Parsol MCX | 1.25 | 1.25 | 1.25 | 1.25 | 1.25 | 1.25 |
| Particle | 0 | 3.00 | 3.00 | 0 | 3.00 | 3.00 |
| Dye | 0 | 0.26 | 0 | 0.26 | 0.26 | 0.26 |
| Water | To 100 | To 100 | To 100 | To 100 | To 100 | To 100 |

Particle in examples 2, 3 and 5 was titanium dioxide of particle size in the 0.1 to 0.25 micron range. This particle scatters about 83% in the wavelength range of 400 to 550 nm with respect to total scatter in the 400 to 700 nm range.

Particle in example 6 was titanium dioxide of average particle size 0.6 micron. This particle scatters about 49% in the wavelength range of 400 to 550 nm with respect to total scatter in the 400 to 700 nm range.

Dye in example 2, 4 and 6 was Chocolate Brown TAS which absorbs about 90% in the wavelength range of 400 to 550 nm with respect to total absorption in the 400 to 700 nm range.

Dye in example 5 was Brilliant Blue FCF which absorbs about 9% in the wavelength range of 400 to 550 nm with respect to total absorption in the 400 to 700 nm range.

The transmittance and reflectance of the various examples 1 to 6 were measured using the following procedure:
Transmittance measurements: Measured amounts (2 mg/cm²) of the compositions were applied on a 3M transpore tape. Diffused transmittance was measured with the help of a spectro-radiometer with a diffuse collector (model ILT-900 R, International Light) and fiber optic light source (Halogen Light source, Leica)
Reflectance measurements: Measured amounts (2 mg/cm²) of the composition were applied on skin-like substrate. The substrate was a 3M transpore tape glued to a brown paper (L* of 57.7, a* of 5.2 and b* of 9.5) whose colour was similar to human skin (L* 56.4, a* of 9.7 and b* of 20.4) for the measurements. Diffused reflectance was measured using a spectrophotometer (model Macbeth-7000 Eye), equipped with integrating sphere.

The data on the photoprotection provided by the composition of examples 1 to 4 are summarized in table 2.

**Table 2**

| % Transmittance | Example 1 | Example 2 | Example 3 | Example 4 |
|---|---|---|---|---|
| 400 nm | 99.6 | 67.0 | 78.2 | 81.0 |
| 450 nm | 99.2 | 68.7 | 85.0 | 80.3 |
| 500 nm | 99.8 | 76.9 | 89.9 | 87.1 |
| 550 nm | 99.6 | 83.9 | 92.0 | 92.1 |
| 600 nm | 99.8 | 89.6 | 93.4 | 96.1 |
| 650 nm | 99.8 | 89.5 | 94.3 | 95.7 |
| 700 nm | 99.8 | 94.1 | 94.5 | 98.7 |

The lower the transmission values, the better is the photoprotection. Thus, the data in table 2 indicates that composition as per the invention (example 2) provides for better photoprotection as compared to composition without both the particle and dye (example 1) and to composition with only the particle (example 3) and to composition with only dye (example 4).

The reflectance spectra from the surface, which is a measure of the appearance was measured for examples 2 to 4 in comparison to the surface without any composition applied on it (Background). The data is summarized in table 3.

**Table 3**

| % reflectance | Background | Example 2 | Example 3 | Example 4 |
|---|---|---|---|---|
| 400 nm | 18.2 | 14.1 | 24.2 | 11.8 |
| 450 nm | 19.0 | 13.7 | 24.0 | 12.0 |
| 500 nm | 21.7 | 17.6 | 25.7 | 15.9 |
| 550 nm | 23.7 | 21.5 | 27.0 | 19.6 |
| 600 nm | 27.0 | 27.4 | 30.0 | 25.2 |
| 650 nm | 36.4 | 34.3 | 39.7 | 33.3 |
| 700 nm | 48.8 | 51.0 | 50.7 | 49.6 |

The spectra which is closest to that of the background is an indication that the skin appearance is closest to the natural colour of the skin. Data in table 3 indicates that composition as per the invention (example 2) provides a skin appearance which is closest to that of natural skin while those of examples 3 and 4 provide an appearance different from natural skin appearance.

### Example 5: Use of a dye outside the scope of the present invention

A composition (example 5) as shown in table 1 was prepared to study the photoprotection provided by using a dye outside the scope of the present invention. The transmittance spectra provided by this sample was compared to that of example 2 (of the invention). The data is shown in table 4.

**Table 4**

| % Transmittance | Example 2 | Example 5 |
|---|---|---|
| 410 nm | 67.0 | 62.0 |
| 450 nm | 68.7 | 83.0 |
| 500 nm | 76.9 | 89.2 |
| 550 nm | 83.9 | 80.8 |
| 600 nm | 89.6 | 57.8 |
| 650 nm | 89.5 | 49.1 |
| 700 nm | 94.1 | 89.7 |

The desired spectra for good photoprotection is to have low transmittance in the blue region (400 - 500 nm) and slightly higher transmittance in the red (600- 650 nm) region. This is provided by example 2 while an opposite effect is seen with example 5.

### Example 6: Use of a particle outside the scope of the present invention

A composition (example 6) as shown in table 1 was prepared to study the effect of using a particle outside the scope of the present invention. The reflectance spectra provided by this sample was compared to that of background skin and example 2. The data is shown in table 5.

**Table 5**

| % reflectance | Background | Example 2 | Example 6 |
|---|---|---|---|
| 400 nm | 18.2 | 14.1 | 16.4 |
| 450 nm | 19.0 | 13.7 | 16.8 |
| 500 nm | 21.7 | 17.6 | 21.8 |
| 550 nm | 23.7 | 21.5 | 27.0 |
| 600 nm | 27.0 | 27.4 | 33.9 |
| 650 nm | 36.4 | 34.3 | 39.6 |
| 700 nm | 48.8 | 51.0 | 55.7 |

The data in table 5 indicates that the composition outside the invention (example 6) provides for skin appearance (as measured by % reflectance) different from that of the background as compared to composition as per invention (example 2).

The invention thus provides for a photoprotective personal care composition that provides desired visible light protection while maintaining the desired natural appearance of the skin.

## Claims

1. A photoprotective personal care composition comprising
(i) 0.5 to 10% of inorganic pigment particles in the size range between 0.015 to 0.4 micrometer which scatter greater than 50% of light in the wavelength range 400 to 550 nm with respect to total scattered light in the range of 400 to 700 nm selected from titanium dioxide or zinc oxide, and
(ii) 0.01 to 0.5% of organic dye which absorbs greater than 50% of light in the wavelength range 400 to 550 nm with respect to total absorbed light in the range of 400 to 700 nm, selected from FDC Red 4, FDC Red 1 or DC Brown.

2. A composition as claimed in claim 1 wherein said inorganic pigment particles are white.

3. A composition as claimed in claim 1 or 2 wherein said composition is a leave-on composition

4. A composition as claimed in any one of the preceding claims comprising 0.1 to 5% by weight tea extract.

5. Composition as claimed in any one of the preceding claims for use in a method for protection of the external surface of a human body against the harmful effects of visible light without altering the skin appearance.

## Patentansprüche

1. Körperpflegezusammensetzung mit Lichtschutz, umfassend
(I) 0,5 bis 10% anorganische Pigmentteilchen im Größenbereich zwischen 0,015 bis 0,4 Mikrometer, welche mehr als 50% Licht in dem Wellenlängenbereich von 400 bis 550 nm streuen, bezogen auf das gesamte gestreute Licht in dem Bereich von 400 bis 700 nm, ausgewählt aus Titandioxid oder Zinkoxid, und
(II) 0,01 bis 0,5% organischen Farbstoff, welcher mehr als 50% Licht in dem Wellenlängenbereich von 400 bis 550 nm absorbiert, bezogen auf das gesamte absorbierte Licht in dem Bereich von 400 bis 700 nm, ausgewählt aus FDC Rot 4, FDC Rot 1 oder DC Braun.

2. Zusammensetzung gemäß Anspruch 1, wobei die anorganischen Pigmentteilchen weiß sind.

3. Zusammensetzung gemäß Anspruch 1 oder 2, wobei die Zusammensetzung eine solche ist, die äußerlich verbleibt.

4. Zusammensetzung gemäß irgendeinem der vorhergehenden Ansprüche, umfassend 0,1 bis 5 Gew.-% Tee-Extrakt.

5. Zusammensetzung gemäß irgendeinem der vorhergehenden Ansprüche zur Verwendung in einem Verfahren zum Schutz der äußeren Oberfläche eines menschlichen Körpers gegen die schädlichen Auswirkungen von sichtbarem Licht, ohne das Aussehen der Haut zu verändern.

## Revendications

1. Composition photoprotectrice de soins personnels comprenant
(i) de 0,5 à 10 % de particules de pigment inorganique dans l'intervalle de taille de 0,015 à 0,4 micromètres qui dispersent plus de 50 % de lumière dans l'intervalle de longueur d'onde de 400 à 550 nm par rapport à la lumière dispersée totale dans l'intervalle de 400 à 700 nm choisies parmi du dioxyde de titane ou de l'oxyde de zinc, et
(ii) de 0,01 à 0,5 % de colorant organique qui absorbe plus de 50 % de lumière dans l'intervalle de longueur d'onde de 400 à 550 nm par rapport à la lumière absorbée totale dans l'intervalle de 400 à 700 nm, choisi parmi le rouge FDC 4, le rouge FDC 1 ou le brun DC.

2. Composition selon la revendication 1, dans laquelle lesdites particules de pigment inorganique sont blanches.

3. Composition selon la revendication 1 ou 2, dans laquelle ladite composition est une composition à laisser poser.

4. Composition selon l'une quelconque des revendications précédentes comprenant de 0,1 à 5 % en poids d'extrait de thé.

5. Composition selon l'une quelconque des revendications précédentes pour une utilisation dans un procédé de protection de la surface externe d'un corps humain contre les effets nocifs de lumière visible sans altération de l'apparence de la peau.
